# EUROPEAN PATENT SPECIFICATION

(11) **EP 2 197 399 B1**
(45) Date of publication and mention of the grant of the patent: **24.07.2013**
(21) Application number: 08758515.4
(22) Date of filing: 14.05.2008
(51) Int. Cl.: A61F 9/008, A61F 9/007, A61M 1/00

(54) **ONE-HAND DEVICE FOR OPHTHALMIC SURGERY**
EINHAND-VORRICHTUNG FÜR DIE OPHTHALMISCHE CHIRURGIE
DISPOSITIF À MANIER D'UNE SEULE MAIN POUR CHIRURGIE OCULAIRE

(30) Priority: 19.09.2007 DE 102007044790
(43) Date of publication of application: 23.06.2010
(73) Proprietor: Mann, Dieter, 63839 Kleinwallstadt (DE); Zeller, Philipp, 8143 Stallikon (CH)
(72) Inventor: Mann, Dieter, 63839 Kleinwallstadt (DE); Zeller, Philipp, 8143 Stallikon (CH)
(74) Representative: Prüfer & Partner GbR European Patent Attorneys
(86) International application number: PCT/EP2008/003867
(87) International publication number: WO 2009/036818

(56) References cited:
- EP-A- 0 864 310
- EP-A- 0 919 210
- WO-A-99/04700
- DE-A1- 19 711 675
- FR-A- 543 848
- US-A- 3 945 375
- US-A- 4 167 943
- US-A- 5 716 363
- US-A- 5 871 492
- US-A1- 2001 023 331
- US-A1- 2002 013 572
- US-A1- 2002 019 607
- US-B1- 6 485 499

## Description

The invention is related to a surgical instrument, which preferably can be used in ophthalmic surgery, as disclosed in independent claim 1.

US 5,871,492 describes a system and a method for reducing and removing an ophthalmic lens of a mammalian eye. The system includes a rotary lens-reducing probe device comprising a tubular outer sheath through which a rotatable drive shaft extends. A rotating lens-reducing head member is positioned on the distal end of the drive shaft.

US 4,167,943 describes a rotatable surgical cutting instrument, wherein the axis of rotation is in parallel to the longitudinal axis of the surgical instrument.

WO 99/04700 describes a method and an apparatus for irrigatingly dethrombulating bladder fluid. In particular, there is a central shaft with dethrombulating blades mounted to it, wherein the central shaft is rotating around an axis that is in parallel to the longitudinal axis of the device.

US 3,945,375 describes an instrument for removing tissue including a rotatable fluted cutter member housed in a probe. The axis of rotation is again in parallel to the longitudinal axis of the device.

In surgical interventions in the eye, in which tissue is taken from the eye, normally the removed material is replaced by a solution for infusion. This is done to uphold the inner pressure in the eye-chamber.

Hitherto a gravity-controlled infusion has been used for this, in which the intraocular pressure corresponds to the infusion pressure that is determined by the height of the bottle unless material is suction-extracted from the eye simultaneously.

When material is suction-extracted from the eye simultaneously, the actual intraocular pressure (IOP) results from the interplay of infusion rate and aspiration rate. Ideally the intraocular pressure will be so high that the normal intraocular pressure is slightly exceeded and the anterior chamber and the posterior chamber are posed well without a prolapsing of intraocular tissue.

When the aspiration opening is closed by tissue particles or when the aspiration pump is stopped, the IOP rises to the infusion pressure that is determined by the height of the bottle. When on the other hand the infusion is obstructed or shut, there is the danger of a decrease of the intraocular pressure till the bulbus collapses. By a change of the height of the bottle, even when applying motor-driven infusion stands, the pressure variations that often occur within split seconds cannot be duly compensated.

In the prior art there are instruments, in which a cutting device for extracting material is arranged at a handpiece, wherein an extraction opening for extracting the cut material by suction is located immediately behind the cutting tool. It is possible to have a design, in which an irrigation liquid or infusion liquid is also supplied via this handpiece. However, thereby the diameter of the tip of the instrument that is introduced into the eye increases. This, however, means that a relatively large opening has to be created through which the instrument can be introduced into the eye. In view of an infection risk, the healing time and possible damages to healthy eye tissue such as corneal astigmatism - which can be induced when suturing the wound - this is disadvantageous. Therefore, normally the irrigation fluid in anterior segment surgery is supplied via a separate infusion needle, which is introduced into the eye bimanually through a separate opening. In posterior segment surgery the infusion needle is sewed into a separate (third) opening.

As already mentioned above, in order to attain a micro-incision surgery, a diameter of the instruments as small as possible has to be aimed at. Therefore, the diameter of the aspiration pipe in the handpiece is not particularly large. This can lead to a plugging of the pipe with cut and sucked off tissue. In such a case, the intraocular pressure (normal pressure around 15 mmHg, approximately 20 cm water column) rises to the bottle pressure and stays at this predetermined height until the aspiration pipe is again open. With a usual bottle height of 65 cm (approximately 48 mmHg) above the eye of the patient this leads to the problem that the intraocular pressure rises to more than three times the normal pressure. Thereby, diverse damages a.o. of the optic nerve can result. Choosing a lower bottle height during the aspiration is disadvantageous, because in this case there is the danger that the eye collapses.

The pressure variations generate physical work that acts on the intraocular structures like the corneal endothelium, the iris, the lens and its capsule by changing the depth of the chamber.

However, an occlusion of the aspiration pipe cannot only be caused by removed material. Also the mode of operation of conventional cutting tools induces this as a result of their operating frequency of opening and closing.

As an example, Fig. 11 shows a cutting tool of the prior art as it is described in US 4,099,529. The instrument consists of an outer hollow tube 911 and an inner hollow tube 913 which is concentrically inserted in it. The outer hollow tube 911 has an aperture 917 laterally to its tip. The aspiration pipe is located inside of the inner hollow tube, so that tissue to be cut at first is sucked by the aperture 917, whereupon the inner hollow tube 913 that can be linearly moved inside of the outer hollow tube 911 moves in such a way that it closes the aperture 917 with its wall. Here a cutting edge is provided at the front end 920 of the inner hollow tube 913, which cutting edge shall cut off the sucked material when closing the aperture (917). It can be seen that in a state, in which the aperture 917 is closed, suddenly the suction of the aspiration pipe is no longer present outside of the instrument and the material to be cut is no longer drawn to the cutting instrument and drops off from it. In this state the intraocular pressure is raised, which arises from an increased depth of the chamber. The pressure immediately decreases again when the inner pipe 913 unblocks the aperture 917, whereby the depth of the chamber becomes smaller. However, during the removal of material by the back and forth movement of the inner hollow (cutting) tube 913 pressure fluctuations are generated. Moreover, for example in vitreous surgery (vitrectomy) the vitreous, which is temporarily sucked and held and temporarily is not sucked, because no vacuum is present, is set into oscillations. These oscillations of the vitreous body may exert a pull on the retina and thereby may provoke a retinal detachment or retinal tears.

In view of the above-described problems of the prior art an object of the present invention is to provide an ophthalmological instrument, in which fluctuations of the intraocular pressure are avoided. At the same time, an instrument shall be provided, which has a smaller diameter of the tip and is easy to handle.

The object is achieved by a one-hand device according to independent claim 1.

Further developments of the invention are specified in the dependent claims.

Further features and advantages of the invention arise from the following description of embodiments based on the figures, of which:
Fig. 1 shows a schematic view of a complete system, in which the device according to the invention is used,
Fig. 2 shows a view of the handpiece according to the invention,
Fig. 3a shows a side view of a tip having a cutting body/milling body integrated at its front end,
Fig. 3b shows a side view of a tip having two irrigation openings, wherein the side view is rotated by 90° with respect to the view of Fig. 3a,
Fig. 3c shows a side view of a tip, in which the cutting body/milling body and the aspiration opening are arranged in the transition area between the front end and the cylindrical wall of the hollow tube,
Fig. 3d shows a side view of a tip having a cutting body/milling body arranged in the cylindrical wall of the hollow tube,
Fig. 3e shows a side view of a tip having more than one aspiration opening,
Figs. 4a to 4c show examples of designs of a cutting body/milling body,
Fig. 5 shows a view of an instrument that is particularly suited for a use in cataract surgery,
Fig. 6a shows an enlarged view of a cutting body/milling body having a facing with disintegration tools,
Fig. 6b shows an enlarged view of a disintegration tool,
Fig. 7a shows the vitrectomy instrument of Fig. 3a with attached optics in the form of a lens,
Fig. 7b shows the vitrectomy instrument of Fig. 3d with attached optics in the form of a prism,
Fig. 8 shows an instrument having a front end that is formed to be an injection needle for a one-step opening of the eye,
Fig. 9 shows a cut at right angle to the longitudinal axis of the tip of Fig. 8,
Fig. 10 shows examples of shapes of the tip and

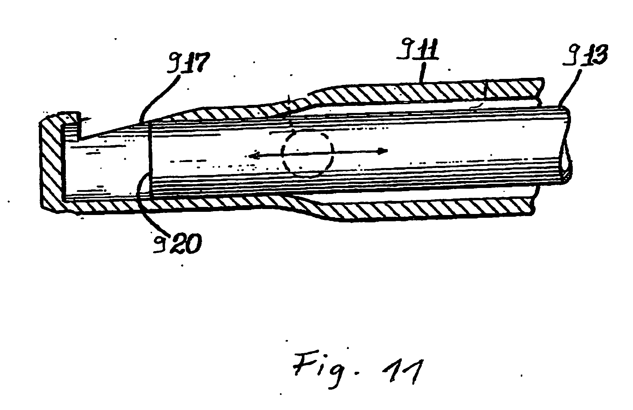

Fig. 11 shows a cutting tool of the prior art.

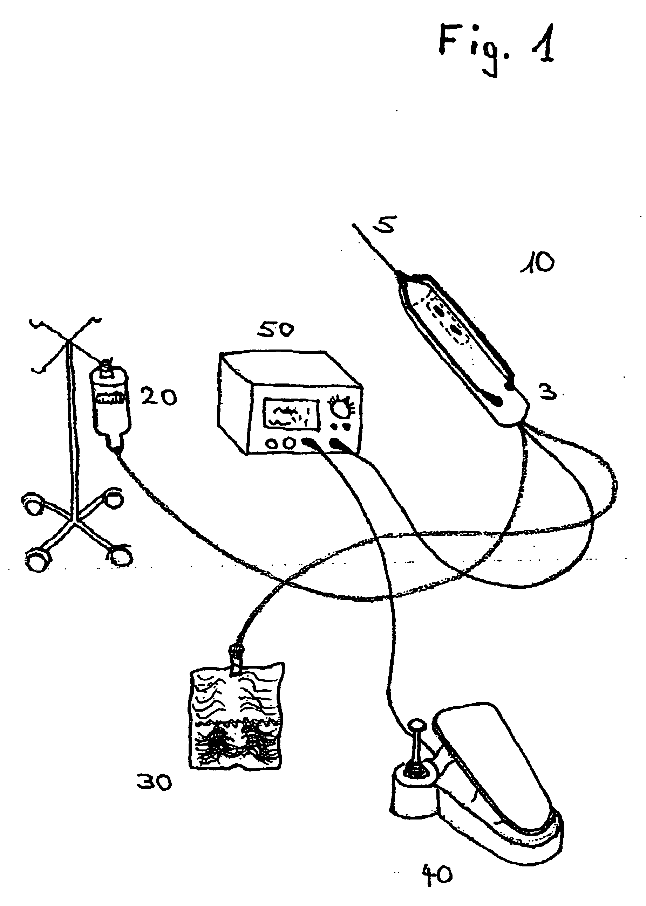

Fig. 1 provides an overview of a complete system, in which the instrument according to the invention can be used. The instrument 10 consists of a handpiece 3, to which a tip 5 is attached that can be introduced into the eye. The infusion solution, in the following referred to as irrigation solution or liquid is supplied from a container or infusion bag 20 to the handpiece 3 and the sucked off or aspirated material is discharged into a container or collection bag 30. A main control unit, at which the individual functions are specified, the current supply is effected using mains operation or low-voltage operation and to which the foot switch is connected, is provided with the reference number 50.

A multi-function foot switch, which can optionally be connected in order to control the instrument, is designated with the reference number 40.

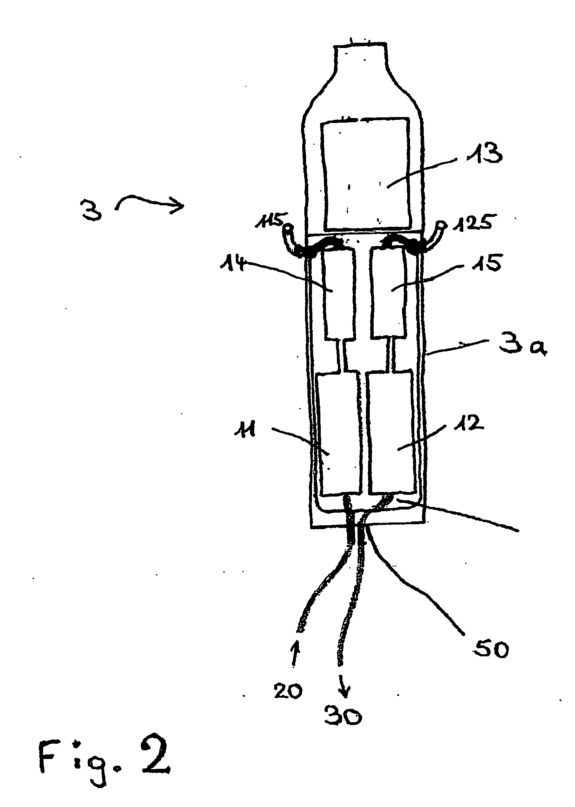

Fig. 2 shows a cross-section of the handpiece 3, which schematically reflects the inner structure. For a better illustration the tip 5 is not shown. However, the ends of an irrigation conduit 115 and of a suction conduit 125, also referred to as aspiration conduit 125 in the following, which extend into the tip 5, can be seen at the handpiece 3 that is formed in the form of the tube 3a. The irrigation conduit 115 and the suction conduit 125 are run to the tip outside of the handpiece. In this way an easy cleaning of the instrument is possible. Such a running of the conduits, however, is not mandatory. Conduits 115 and 125 running to the tip inside of the tube 3a would also be conceivable provided that a good cleaning of the instrument is possible.

The irrigation conduit is connected to an irrigation fluid pump 11 that is arranged in the tube 3a. Furthermore, an irrigation fluid sensor 14 is shown in the irrigation conduit 115 between the upper end of the tube 3a and the irrigation fluid pump 11, which irrigation fluid sensor may be, for example, a flow rate meter or a pressure sensor. In the same way the aspiration conduit 125 is connected to a suction pump or aspiration pump 12 that is also arranged in the tube 3a. Also, an aspiration fluid sensor 15 is provided between the aspiration pump 12 and the upper end of the tube 3a.

By the described setup the irrigation fluid pump 11 is arranged between the irrigation conduit 115 and the container/infusion bag 20 for the irrigation fluid. Also, the mentioned aspiration pump 12 is arranged between the aspiration conduit 125 and the container/collection bag 30 for the aspirated material.

The flow rate through the irrigation conduit 115 is adjusted to the flow rate through the aspiration conduit 125 by means of a control 13 that may, for example, be also accommodated in the handpiece 3, however, can also be arranged outside thereof in a main control unit 50. The goal of the control is an active regulation of the intraocular pressure and of the flow rate during the whole duration of the surgery. To this effect the outputs of the two sensors 14, 15 are transferred to the control 13, which then adjusts the flow rate through the irrigation conduit. Here, the regulation of the flow rate can be effected by regulating the pumping capacity of the irrigation fluid pump 11. However, optionally a flow rate regulator 115a can also be arranged in the irrigation conduit, which flow rate regulator 115a then is suitably controlled by the control 13.

The measurement results of the sensors 14 and 15 are the basis for the regulation. If these sensors 14 and 15 for example provide values for the flow rates through the irrigation conduit 115 and the aspiration conduit 125, then the control 13 may intervene in a regulating way until both measurement values are equal. When the sensors 14, 15 are pressure sensors, then the control determines the actual flow rate in the irrigation conduit 115 and the aspiration conduit 125 taking into account the ratios of cross-sections in the region of the sensors 14, 15 and uses them as basis for the regulation.

The flow rate in the aspiration conduit can be set directly by the operator.

According to the present invention the flow rate of the irrigation fluid is actively regulated in dependence of the flow rate in the aspiration conduit 125 and/or the intraocular pressure. Thereby the system is resistant against an occlusion or clogging of the aspiration conduit 125. If such an occlusion did occur, the control 13 would automatically throttle the flow rate in the irrigation conduit, which may also include a stoppage of the irrigation fluid pump 11 or an inversion of its pumping direction, so that no undesired overpressure may occur inside of the eye.

A further advantage with respect to the prior art results from the independence of the system according to the invention on the height of the container/infusion bag with the irrigation solution relative to the patient's eye. Thereby a change of height of the operating table and positional changes of the patient are possible without any problems.

The tip 5, in which one, two or a plurality of openings 5a for the exit of the irrigation fluid and one or a plurality of aspiration openings 5b for holding the sucked material and for taking up material to be sucked off are located, can be fixedly connected to the tube 3. However, it is also possible to choose an embodiment in which the tip 5 can be removed and in which the tip 5 is for example attached to the handpiece 3 by means of a plug connection, a screw coupling, a ball catch, taper catch or a ring detent or a bayonet mount. In this way it is easier to access the inside portions of the tube 3a and the tip 5 for a cleaning, disinfection and sterilization. Preferably the tip 5 is designed as disposable for a one-time use.

The arrangement of the pumps in the handpiece 3 leads to short pipes, so that the distance to the openings 5a and 5b is not large. Thereby a quick reaction to pressure fluctuations is possible compared to a hydrostatic system, in which the height of the infusion container/bottle can be changed or aseptic air may be supplied to the infusion bottle. Due to the length of the pipes in such a hydrostatic system only a slow reaction to pressure fluctuations is possible. The short length of the pipes in the system invention in addition makes easier the sterilizability, for example by autoclaving. Preferably, for this the instrument 10 or the handpiece 3 is removed from the complete system and separately cleaned.

The pumps 11, 12 are extremely precise and are not vulnerable to counter-pressure. For instance, gear micro pumps are suitable as pumps 11, 12. In these pumps a direction of rotation in both ways is possible, whereby pressure fluctuations can be effectively counteracted by changing the pumping direction.

It is possible to use the handpiece 3 with various tips 5. Here in the first place one can think of tips having a cutting tool/milling tool for removing material. Cutting tools that are known in the prior art are suitable. However, it is also possible to integrate a novel cutting tool/milling tool according to the invention, which, by itself alone without a combination with the above handpiece 3 according to the invention, can lead to a reduction of the pressure variations and is described in the following.

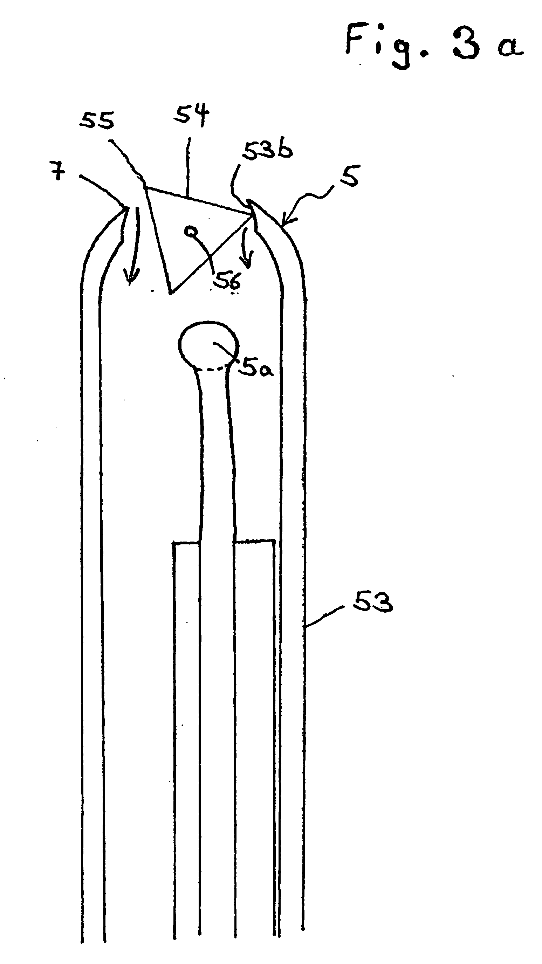

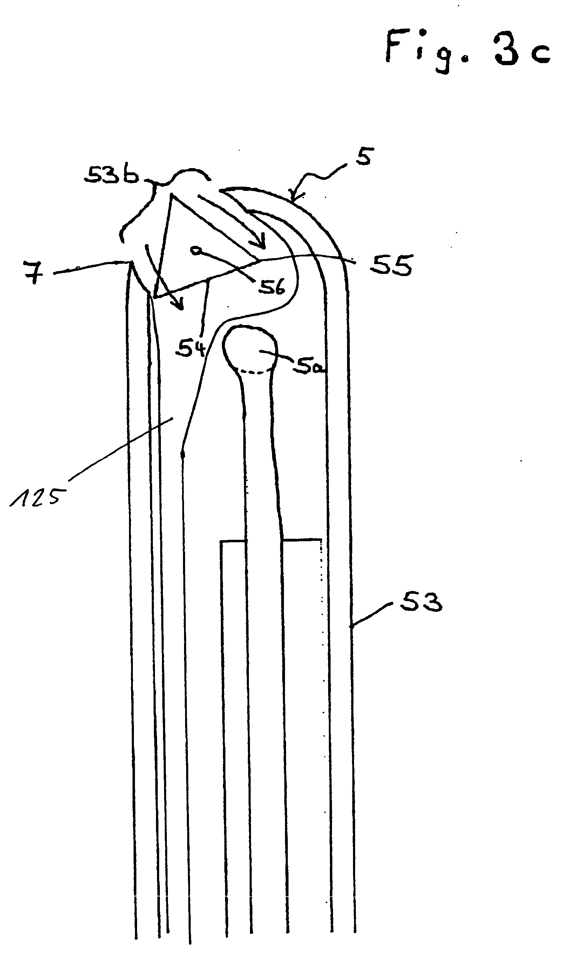

Figs. 3a to 3d in this connection show as an example a tip 5, in which a cutting tool/milling tool 54 is inserted. As is shown in Figs. 3a to 3d, the tip 5 consists of a cylindrical tube 53 having a longitudinal axis and a cross-section that is not necessarily circular. An aspiration opening 53b is arranged in the front part of the tube 53 and inside of the tube 53 an aspiration conduit or channel 125 that is connected to the aspiration opening 53b is present, which aspiration conduit is explicitly shown only in Fig. 3c. Immediately behind the aspiration opening 53b a cylindrical cutting body/milling body 54 is pivot-mounted inside of the tube 53.

In the present case the cylindrical cutting body/milling body 54 has the shape of a prism with a triangular base. The cutting body/milling body 54 has a plurality of seperating edges or severing edges 55, which are sharp, so that they are suited for cutting. An axis of rotation 56 passes through the base plane and the top plane of the cutting body/milling body 54 such that it is close to one of its principal axes of inertia or even coincides with it. In the figures the axis of rotation 56 is perpendicular to the longitudinal axis of the tube 53. However, also a different orientation in space is possible. Preferably the position of the axis of rotation 56 is set such that it is in parallel or nearly in parallel to the tangent to the imaginary continuation of the peripheral surface of the cylindrical tube in the aspiration opening 53b (also simply described as 'tangential to the aspiration opening').

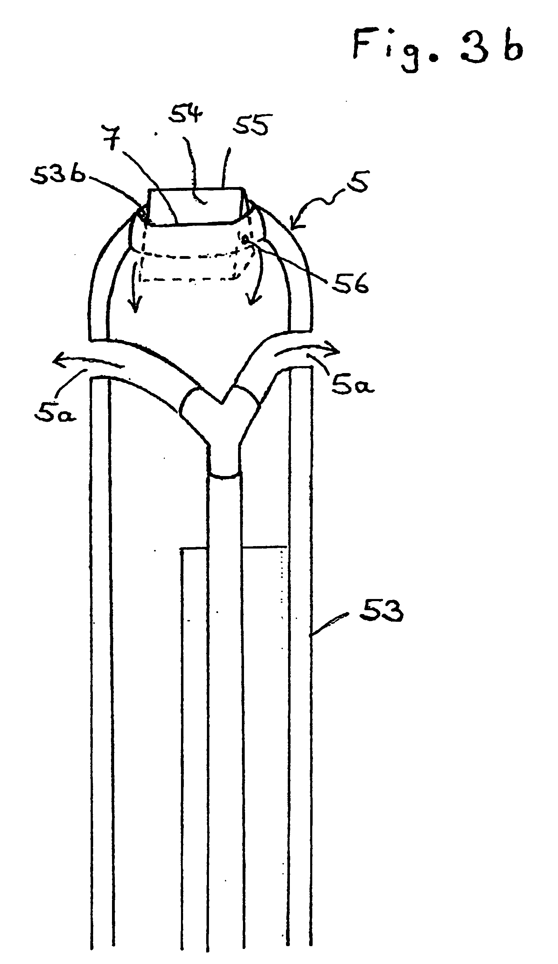

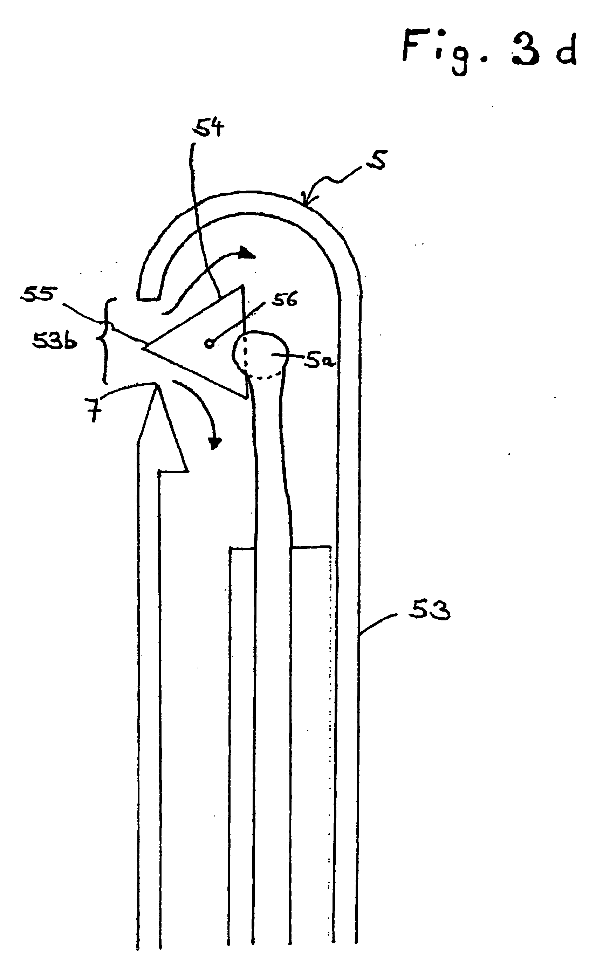

The tip having a lateral aspiration opening in the tube 53, which is shown in Fig. 3d, is in particular suited for performing an anterior segment surgery or a posterior segment surgery. By arranging the cutting body/milling body 54 such that the axis of rotation 56 is tangential to the aspiration opening 53b, it is possible to arrange the cutting body/milling body 54 closer to the front end of the tip 5. Thereby it becomes possible to approach the retina very closely during interventions at the vitreous. Accordingly, in Figs. 3a and 3b embodiments are shown, in which the cutting body/milling body 54 is arranged directly at the front end. In order to avoid injuries of the retina and in order to visualize the aspiration opening a compromise could also be an arrangement of the cutting body/milling body 54 in the transition area between the cylindrical tube wall and the front end, as it is shown in Fig. 3c.

The at least one irrigation opening can also be arranged either laterally in the wall of the tube 53 or else may be arranged closer to the front end of the tip 5 and directly at the front end, respectively.

The sizes and the positions of the cutting body/milling body 54 and the aspiration opening 53b are matched to each other such that at the left side and at the right side of the cutting body/milling body 54 there remains a clearance to the edge of the suction opening 53b, which is illustrated in Figs. 3a to 3d by two arrows. Thereby the material to be removed is sucked into the clearances that remain between the cutting body/milling body 54 and the suction opening 53b and held there. Thereby the material between the edge of the opening 53b and the cutting edges or separating edges is cut due to the rotary movement of the cutting body/milling body 54.

Compared to the cutting tools of the prior art, in which the whole aspiration conduit is completely closed in a cutting procedure, in the present invention the material can be sucked at other positions (for example a clearance between the cutting body/milling body 54 and the edge of the aspiration opening that is far from the separating edge) even during the cutting procedure, in which a separating edge 55 is exactly opposite to the edge of the aspiration opening 53b. Thereby the suction procedure is not interrupted and pressure fluctuations are avoided. Even if the aspiration conduit is completely closed, the pressure variations will be still smaller than in the prior art, because the cutting frequency is considerably higher due to the plurality of separating edges at the periphery of the cutting body/milling body. Moreover, a separating edge and the edge of the aspiration opening lie opposite to each other only for a very short time. Contrary to the prior art, the suction takes place outside of the cutting body. For this the aspiration conduit 125 is run to the aspiration opening 53b outside of the cutting body/milling body 54, so that it is connected to the outside of the tube 53 via the space or clearance between the outer side of the cutting body/milling body 54 and the edge of the aspiration opening 53b. This clearance, however, is closed only for a very short period, during which the separating edge and the edge of the aspiration opening exactly lie opposite to each other. As both edges are very narrow, the suction action is immediately available again as soon as both edges are no longer lying exactly opposite to one another.

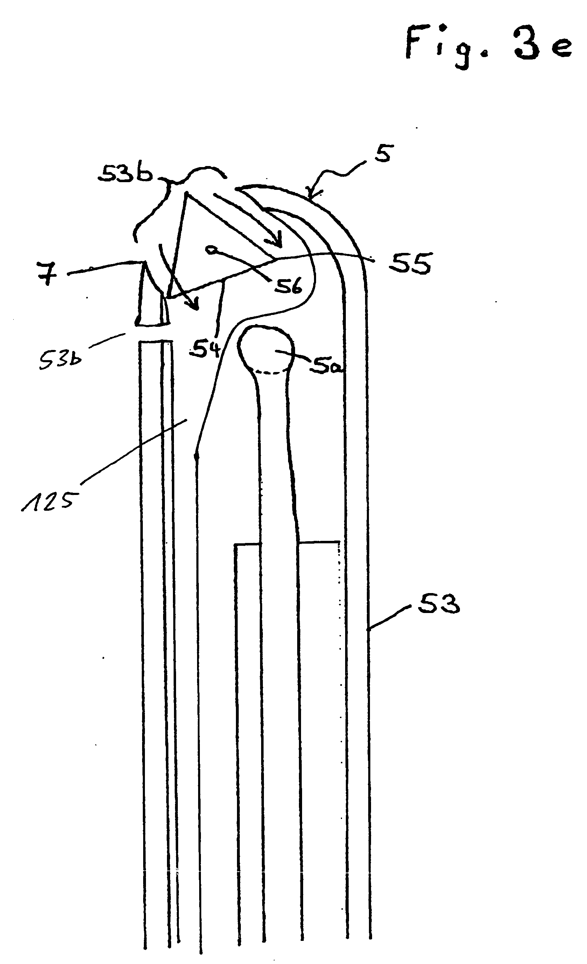

Moreover, further aspiration openings 53b may be systematically added in the tube 53 nearby the cutting body/milling body 54, wherein the tissue is sucked by means of these further aspiration openings. This is exemplified in Fig. 3e. By the very small cross-section of these openings and due to the absence of a cutting body/milling body in these openings they are at no time closed by material that is to be removed. They are merely further suction points for fixing the material.

For instance in a vitrectomy the vitreous material is separated or cut off by a co-action of a separating edge 55 and an edge 7 of the suction opening 53b that acts as counter-edge. In the process the cutting edge and the tissue are subject of a constant pressure. Thereby a sharp dissection is enabled. The cutting edge need not necessarily be located at the cutting body/milling body 54. Rather, it is also possible to form the cutting edge at the counter-edge 7 at the rim of the suction opening 53b, where the separating edges 55 merely press the material against the cutting edge at the counter-edge 7. In addition, cutting edges may be formed both at the cutting body/milling body 54 and the counter-edge.

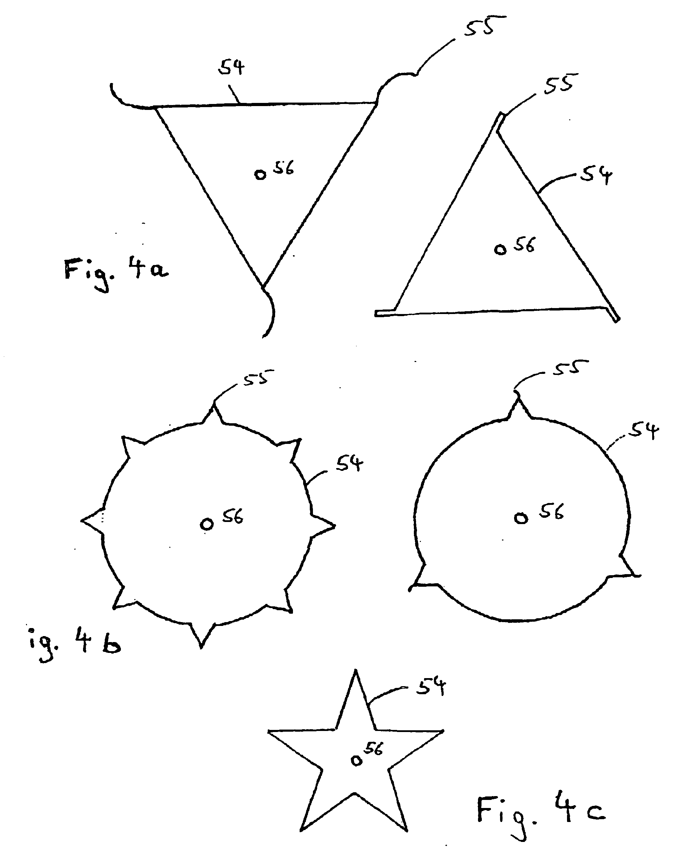

The seperating edges 55 or severing edges 55 at the cutting body/milling body 54 need not necessarily coincide with a geometrical edge of the cutting body/milling body 54. As is illustrated in Fig. 4a, it is also possible to form vane-shaped appendices at the geometrical edges, wherein the severing edges or cutting edges 55 are formed at the ends of the vane-shaped appendices. Preferably the severing edges 55 or cutting edges 55 should be arranged at the periphery of the cutting body/milling body 54 with equal distances to one another.

The term "cylinder" is used in the present application according to its general geometrical definition according to which it includes bodies that have an arbitrarily shaped plane base and top surface, which can be made to overlap completely when being shifted along parallel straight lines. As is shown in Fig. 4b, the cutting body/milling body 54, of course, can also be designed as cylinder having a circular surface, wherein severing edges/cutting edges 55 may be attached at the periphery of the cylinder, which severing edges/cutting edges 55 may alternatively also be located on vane-shaped appendices. In particular a cylinder having a star-shaped base like in Fig. 4c is also conceivable. Further, a triangular prism base is not a basic requirement for a proper function. Rather, prisms in which the base is bordered by an arbitrary polygonal chain, may be used.

Furthermore, it is also possible to design the cutting body/milling body 54 in the shape of a cone, truncated cone, a pyramid, a truncated pyramid, an ellipsoid or as arbitrary solid of revolution. The shape of a spherical segment (portion of a sphere cut off by two parallel planes) in which the severing edges are placed on the lateral surface (zone) of the spherical segment and thereby have a small curvature is also conceivable.

Furthermore, in all previously described geometrical shapes of the cutting body/milling body 54 it may have any convex or concave shape of the base surface and the top surface, respectively. Here, the bordering rim at the edge of the base and the top surface, respectively, can be partially or completely rounded.

A cutting body/milling body 54 that consists of a stack of two or more cylinders is also applicable, wherein the severing edges 55 of two adjacent cylinders of the stack are angularly displaced against one another. Finally, the cutting body/milling body 54 may have at its periphery arbitrary impressions, in particular also gaps perpendicular to its axis of rotation 56 as well as through-holes.

Ultimately not even the whole cutting body/milling body 54 need have severing edges 55 at its peripheral surface. It is sufficient to have the severing edges 55 merely in that part of the peripheral surface of the cutting body/milling body 54 that is exposed in the region of the aspiration opening 53b. All above specifications concerning the shape of the cutting body/milling body 54 and the design of the severing edges in this case need only apply for that part of the cutting body/milling body 54 that has the severing edges. However, care must be taken that as a result of asymmetries the unbalances during the rotation do not get too large.

Moreover, the cutting body/milling body may have such a configuration that at least one portion of it is hollow or merely consists of a holder for the rotating severing edges. Then the body structure looks like an agitator. A design, in which at least one portion of the cutting body/milling body has the shape of a curved surface, is also conceivable. For this the cutting body/milling body is for example designed such that a material layer such as a sheet is bent to have the shape of the letter "S". The axis of rotation then coincides with the symmetry axis of the body thus generated, wherein the severing edges correspond to the edges of the sheet that are not curved. It can be seen that based on this design also a vane wheel having more than two severing edges is suited as cutting body/milling body.

In summary, the cutting body/milling body 54 may have a multitude of shapes in particular also hybrids of the above-mentioned geometrical bodies, as long as it is ensured that the respective lateral surface having the severing edges 55 is nearly tangential to the aspiration opening 53b when it is exposed in the region of the corresponding aspiration opening 53b. In particular, when the cutting body/milling body has hollows, one can think of creating a connection between these hollows and the aspiration conduit. However, also in this case it is important that a part of the aspiration is effected via the clearance between the cutting body/milling body 54 and the edge of the aspiration opening 53b.

The cutting body/milling body 54 can be made from a metal such as stainless steel or titanium or from ceramics. The cutting edges preferably are coated with diamond. However, also other hard materials such as zircon, corundum, an Si ceramics or oxide ceramics or metal are suited. Furthermore, it is possible to form toothed cutting edges 55.

The drive of the cutting body/milling body 54 can for example be effected by means of a motor that is accommodated in the handpiece 3. For example, a magnet can be integrated in the cutting body/milling body 54, which magnet is driven via an electromagnetic loop in the tip 5 like a classical electric motor. For this preferably two small electromagnets are provided in the sidewall of the tip. Here, the feed cables for the magnets can be accommodated inside of the tube 53.

Maximum freedom for an operation of the cutting body/milling body 54 is achieved, when the cutting body/milling body 54 can rotate in both directions and can also operate in an oscillating manner. Preferably, it should further be possible to drive the motor such that single cuts and cuts in series may be performed and any severing edge 55 does not protrude from the aspiration opening 53b in a non-operative condition, so that in the inactive state the possibility of injuries of the wound edges when entering the eye is eliminated.

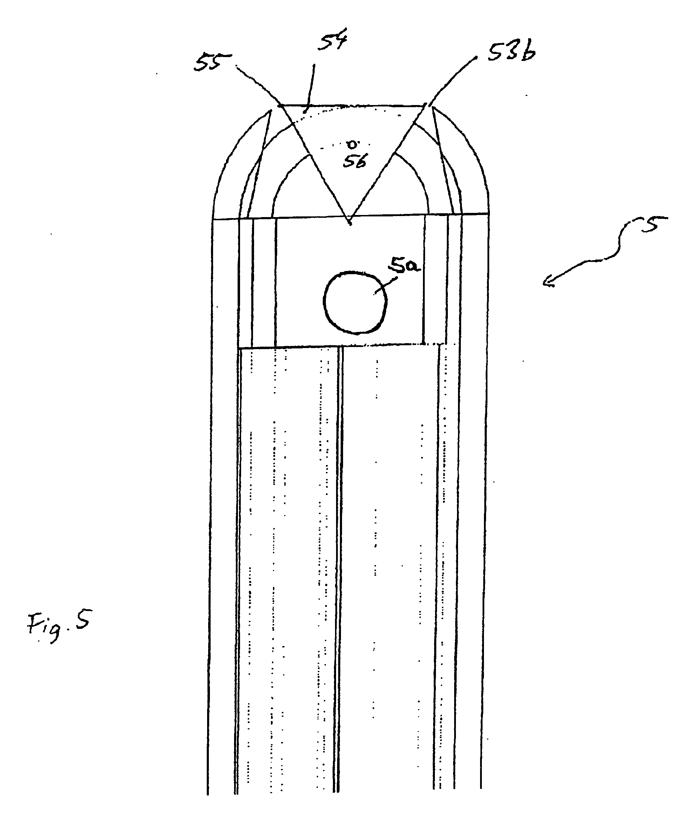

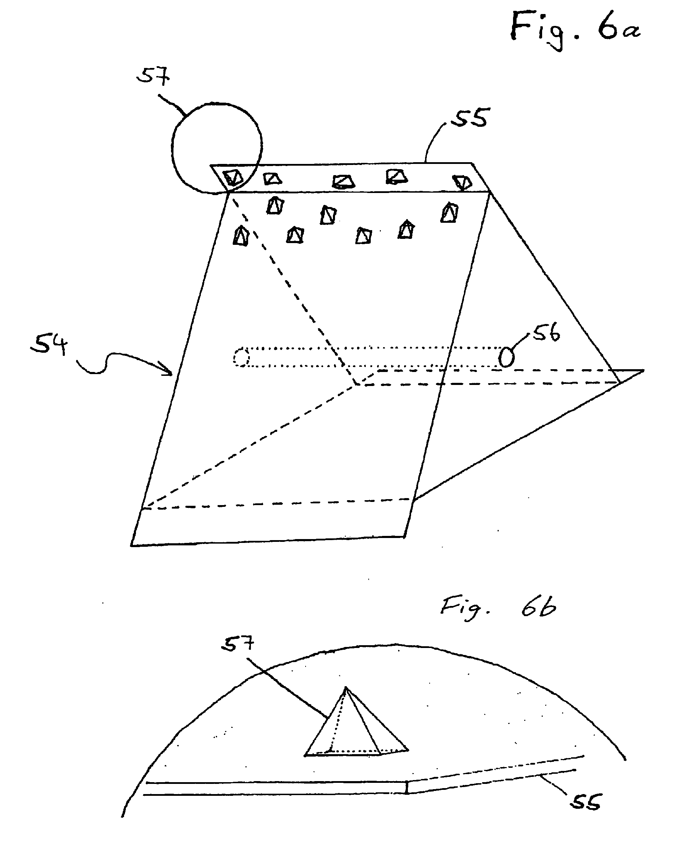

Fig. 5 shows a tip 5, in which the cutting body/milling body 54 is arranged at the front end of the tip together with the aspiration opening 53b. Such a tip is particularly suited for cataract surgery, in which in this way the clouded human lens is removed. For this disintegration tools 57 are fixed to the rotating severing edges 55, as is illustrated in Figs. 6a and 6b. By severing edges designed in this way a disintegration of the lens material is possible. It is possible to change in addition the direction of rotation periodically and to work in an oscillating way or to perform a single cut.

An identical arrangement of the disintegration tools 57 can be chosen for all severing edges 55. However, concerning an areal removal that is as uniform as possible it is advantageous when the respective arrangement of the disintegration tools 57 varies from severing edge to severing edge.

The material of the disintegration tools 57, which tools can also be fixed to the lateral surface of the cutting body/milling body 54 as shown in Fig. 6a, is not limited to diamond that is (epitaxially) grown or applied as coating. It is also possible to use other materials such as zircon, corundum, Si ceramics or oxide ceramics or metal. For the cutting body/milling body having the disintegration tools 57 the same selection of materials is possible like for the cutting body/milling body 54 that lacks these disintegration tools 57.

Compared to a conventional removal of the lens by means of ultrasound due to the mechanical approach the advantage arises that the integrated aspiration also takes over the cooling that is possibly necessary. In addition, the wound-edges at the penetration opening of the instrument are treated with care, which wound-edges can heat up very much in the case of using ultrasound due to the lateral oscillations of the tip (28 or 40 kHz). A pulsation as used when applying ultrasound is not necessary for the mechanical removal.

All tools for removing tissue that were described above have the advantage, which was mentioned above, that the fluctuations of the intraocular pressure are reduced, which fluctuations occur in the conventional irrigation with simultaneous aspiration. By the configuration of the tool according to the invention the cutting body/milling body 54 can freely rotate without that the aspiration opening 53b is closed at any moment. Thereby it is ensured that at each moment a sufficient suction effect is present, so that pressure fluctuations due to the cutting movements are prevented. The faster the cutting body/milling body 54 rotates, the shorter the time the surgeon works intraocularly.

Further, in the tool according to the invention the removal can be dosed more precisely. For example, in a use as vitrectomy instrument the interaction with the counter-cutting edge (counter-cutting edge, cutting edge and tissue are subjected to a constant pressure, which is the physical pre-condition for a sharp dissection) resembles more a cutting process than it is the case for the prior art, where more likely the material is yanked or torn or the material is chopped off. Also, the amount of material removal can be adjusted in a simple way by adjusting the rotation speed of the cutting body/milling body 54. When using the instrument in a lens surgery no large suction is necessary due to the finer dosed removal. Thereby the danger of a rupture of the capsule is reduced.

In the present invention the position of the axis of rotation of the cutting body/milling body 54 does not coincide with a longitudinal axis (center axis) of the tube 53, but is tilted with respect to the longitudinal axis or, is perpendicular to it. Thereby, the aspiration conduit need not be run through the inside of the cutting body/milling body 54, but can be located outside of the same. However, thereby room is made in the tube 53 for further devices in the tip 5 besides the cutting tool:
For instance, glass fibres may be inserted in the walls of the tube 53, which fibres end at the front end of the tip 5 in order to illuminate the surgical zone. Furthermore, an LED may be fitted in the tube 53 or the handpiece 3.

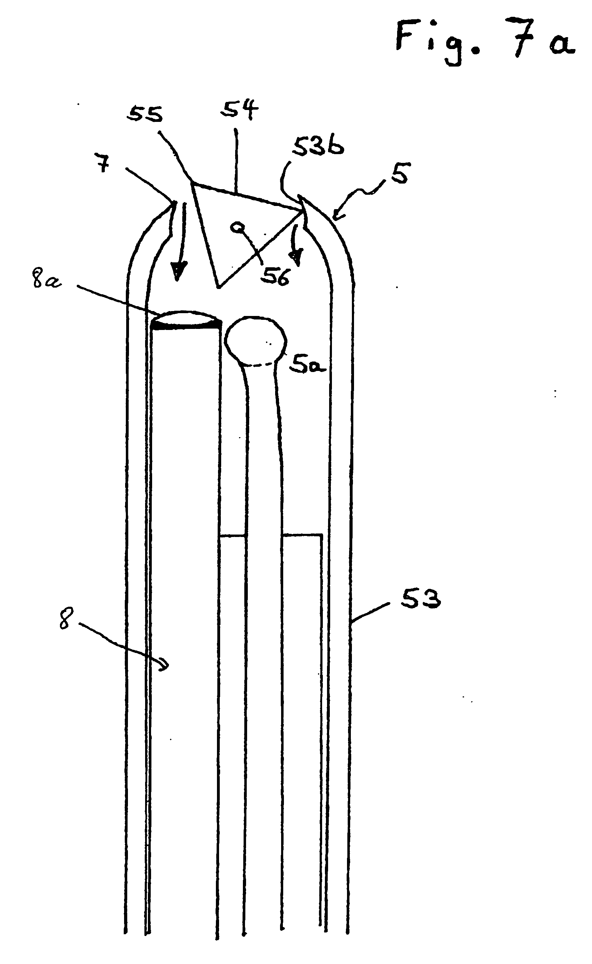

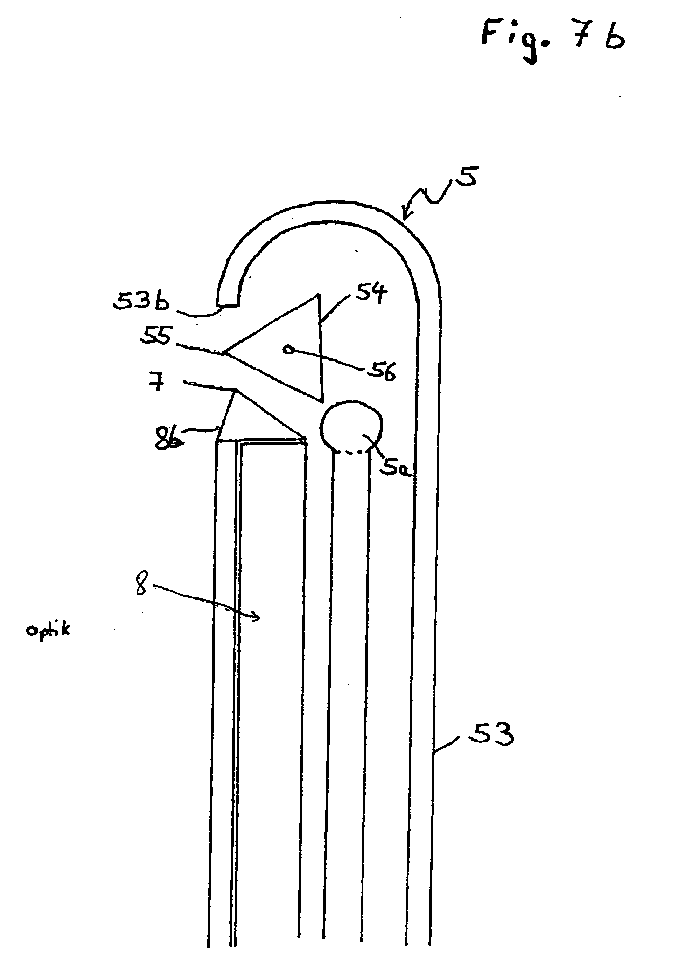

Figs. 7a and 7b show a tip, in which an optics 8 is fitted close to the cutting tool. The optics 8, e.g. an image fibre bundle, serves for visually supervising the surgical field.

At the distal end of the optics 8 for example an optical prism 8b may be positioned, as it is shown in Fig. 7b, a lens 8a, as it is shown in Fig. 7a, or only the polished end of the fibre bundle. Here, the optical prism can have any angle for an optimal access to the field of view. Also, the optical prism itself may comprise the counter-cutting edge 7.

Furthermore, it is possible to run an optical waveguide for a laser up to the front end of the tip. Such a tip then can be used for an endocoagulation during the vitrectomy.

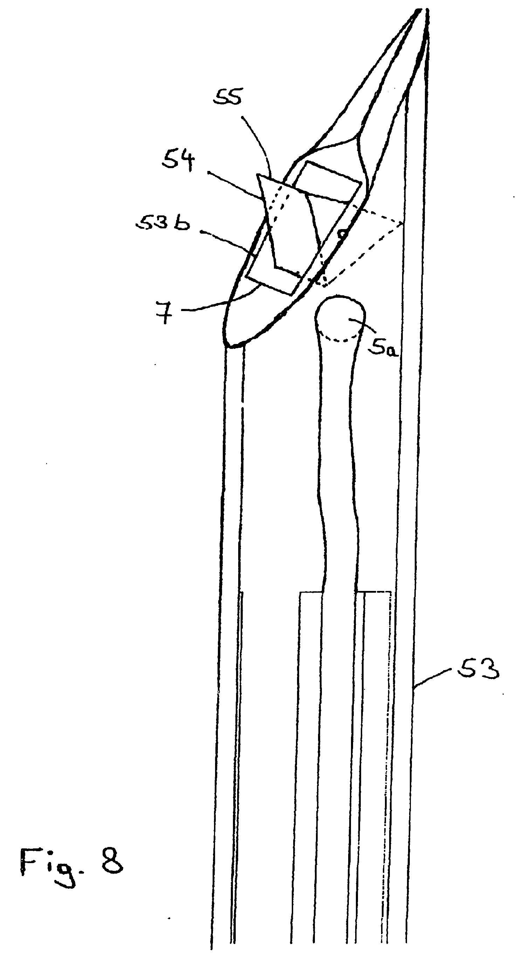

A further field of application opens up when the end of the tip 5 has the shape of an injection needle, as it is shown in Fig. 8. For this the front end of the tip 5 is tapered like a needle. In particular, in Fig. 8 the front end of the tip is bevelled. In this way the slender instrument (diameter 20 or 23GA) can be used for a biopsy with the particular advantage that all functions are provided through one single opening in the eye: a cutting function by the cutting body/milling body 54, a suction function, the irrigation through at least one irrigation opening 5a, optionally an illumination and a monitoring by means of an optics 8 and moreover the use of a laser. The instrument can even be used for applying drugs in the eye in a controlled way. These drugs then are released via the irrigation opening(s) 5a. In particular when the axis of rotation 56 is chosen to be perpendicular to the longitudinal axis of the tube 53 and when the cutting body/milling body is arranged in the bevelled portion, as it is shown in Fig. 8, this is particularly advantageous.

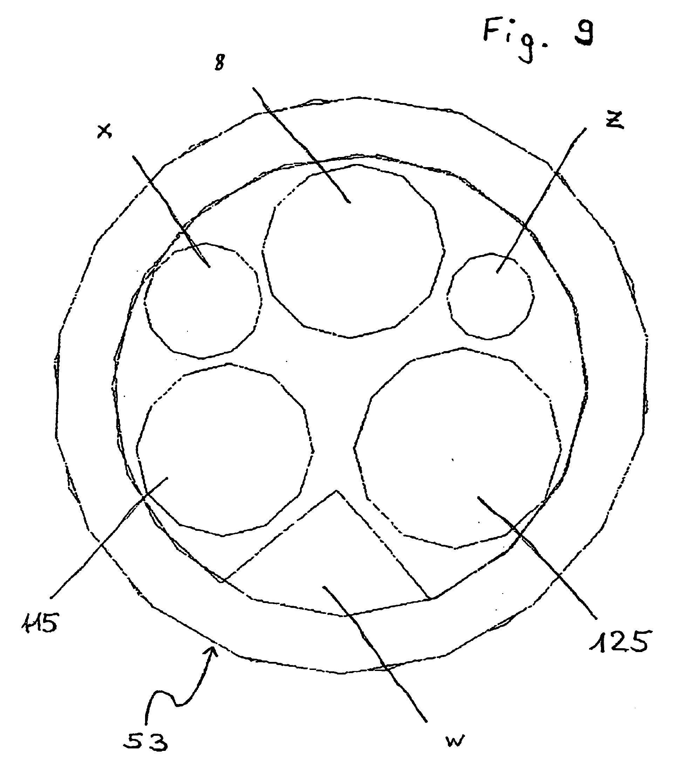

The sectional view of Fig. 9 at right angle to the longitudinal axis of the tip illustrates the accommodation of the diverse functions in the tip 5. There the reference sign W designates a channel for a power supply for the motor for rotating the cutting body/milling body 54, X designates the laser fibre and Z designates the feed line for an illumination of the surgery field (e.g. one or more glass fibres).

Furthermore, the instrument can be operated with one hand using a three-point support, whereby the transmission of force to the tissue can be fine tuned, because the finger tips do not fulfil a holding task and the sense of touch is maintained (Weber-Fechner Law). In the prior art it is necessary to work bimanually with more than one opening in the eye.

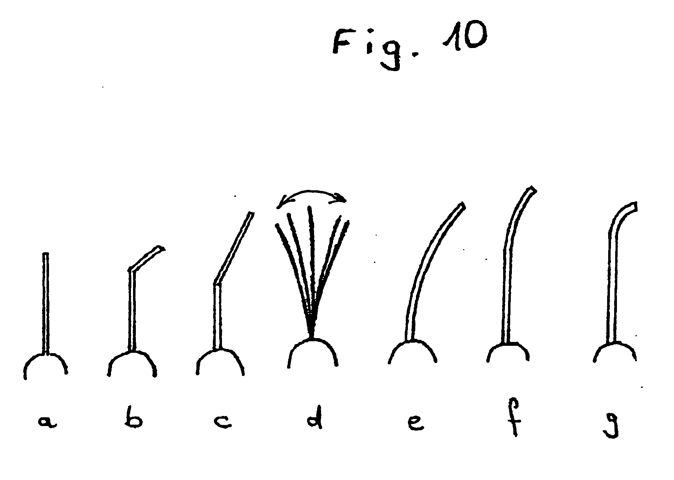

Though in most of the illustrations the tip is shown as straight hollow cylinder, the effects of the present invention are also achieved with curved tips. Examples for possible shapes of the tip 5 are shown in Fig. 10. Fig. 10a shows a rigid, straight tip, Fig. 10b shows a tip that is angled by 45°, Fig. 10c shows a tip that is angled by 30°, Fig. 10e shows a bent tip and Figs. 10f and 10g show tips, in which a section is bent. Bent tips in particular make it possible in an intervention to further advance into the periphery of the eye, wherein the danger of touching the lens is reduced. Here in particular an arrangement of the cutting body/milling body 54 close to the front end of the tip as shown in Figs. 3a to 3c is advantageous.

Fig. 10d illustrates a flexible tip. A one-hand device having such a tip can for example be used in the lacrimal apparatus, in particular in a lacrimal duct, by introducing it "round the corner and the edge, respectively" into the lacrimal sack through the puncta lacrimalis in the lower eyelid.By means of the cutting mechanism of the vitrectomy tip stenoses in the tear drainage ways can be removed. In the endonasal use in particular the fibrous occlusion of the opening of the bone after a dacryocytorhinotomy can be removed. In particular in this application there is the advantage that due to the limited dimensions of the cutting body there is enough space in the tip for integrating an optics, so that the instrument can be applied as endoscope.

Though in the embodiments shown in the figures only one aspiration opening 53b and one cutting body/milling body 54 are shown, also several aspiration openings 53b can be provided at the tip 5, to each of which a cutting body/milling body 54 is assigned. In some cases the tissue can be removed more uniformly due to a plurality of cutting bodies/milling bodies. When the cutting bodies/milling bodies 54 rotate in opposite directions of rotation, a neutralization of the angular momentum is possible, so that it is easier to control the instrument.

In a modification that goes even further a plurality of cutting bodies/milling bodies 54 is provided in one aspiration opening 53b. For instance for two cutting bodies/milling bodies having adjoining axes of rotation it becomes thereby possible to suck the tissue to be cut between the two cutting bodies/milling bodies and not at the edge of the aspiration opening. In this way the cutting action takes place between two separating edges or severing edges 55 that are not located on the same cutting body/milling body. Accordingly, during the cutting process these two separating edges move towards each other due to their rotations. Possibly also an interpenetration of these two separating edges 55 during the cutting action is possible. The just mentioned arrangement can, of course, be also applied to more than two cutting bodies/milling bodies 54.

An arrangement of the cutting body/milling body 54 having the same axis of rotation 56 in one and the same aspiration opening enables again a neutralization of the angular momentum.

Though up to now only applications in ophthalmic surgery have been described, the described instruments are in the same way applicable also in other fields of surgery. Of course, depending on the specific use possibly a change of the dimensions is necessary.

Finally, it should be noted that the described one-hand device, of course, is not limited in use to human medicine. Also a use in veterinary medicine is possible. For this the tip that is used must have larger geometrical dimensions in particular for the vitrectomy of large animals such as in the removal of leptospira of horses. Instead of a length of the tip (5) or approximately 35 mm such as in human medicine the tip length for horses must be approximately 65 mm. The diameter of the tip (5) is also larger. It can be up to 2.0 to 2.2 mm.

## Claims

1. Surgical one-hand device comprising a tube (3) having a tip (5) at its front end, wherein a device for removing body tissue in a surgical intervention is used as said tip,
said device having:
a tube (53) having a longitudinal axis, the tube further having an aspiration conduit (125) and an aspiration opening (53b) for taking up the removed tissue,
a cutting body/milling body (54) that is mounted inside of the tube such that it can rotate around an axis of rotation (56), wherein the cutting body/milling body (54) at least at a part of its periphery has a plurality of separation edges (55),
wherein the cutting body/milling body (54) is mounted in such a way that the separating edges (55) are successively exposed in the aspiration opening (53b) towards the outside of the tube (53) such that they are nearly tangential to the aspiration opening (53b), when the cutting body/milling body (54) rotates around the axis of rotation (56)
wherein the aspiration conduit (125) is run to the aspiration opening (53b) outside of the cutting body/milling body (54).
wherein the tip (5) has at least one irrigation fluid orifice outlet (5a) for the exit of an irrigation fluid
wherein an irrigation fluid orifice outlet (5a) for the exit of the irrigation fluid is provided in the tube (53) of the device for removing body tissue and wherein the one-hand device further comprises
an irrigation fluid pump (11) which is attached at the tube (3) and is connected to the irrigation fluid orifice outlet (5a) via an irrigation conduit (115),
an aspiration pump (12) which is arranged at the tube (3) and is connected to the aspiration opening (5b) via said aspiration conduit (125), **characterized in that** said axis of rotation(56) is tilted with respect to said longitudinal axis of the tube (53) or perpendicular to it, and
an irrigation fluid sensor (14) is arranged at the irrigation conduit (115),
an aspiration fluid sensor (15) is arranged at the aspiration conduit (125) and
said one-hand device comprises a control (13), which
actively regulates the intraocular pressure depending on the measurement results of the sensors (14, 15).

2. One-hand device according to claim 1, in which light can be guided into the tip (5) via an optical light guide.

3. One-hand device according to one of claim 1 to 2, in which an optics is provided in the tip (5) that allows the observation of the surgical field.

4. One-hand device according to one of claims 1 to 3, , in which the cutting body/milling body (54) has the shape of a prism.

5. Device according to one of claims 1 to 4, in which the cutting body/milling body (54) has vanes at the peripheral surface, which are curved in the direction of rotation, wherein the outer edges of the vanes form the separating edges (55).

6. Device according to one of claims 1 to 5, wherein the severing edges (55) are in parallel to the peripheral surface of the cylindrical cutting body/milling body (54).

7. Device according to one of claims 1 to 6, in which at least one separating edge has disintegration tools.

8. Device according to one of claims 1 to 7, in which at the border of the aspiration opening (53b) a counter-cutting edge (7) is formed.

9. Device according to one of claims 1 to 8, in which the separating edge or the disintegration tools or the counter-cutting edge is/are made from stainless steel, zircon or ceramics material.

10. Device according to claim 9, in which diamond crystals are grown on the separating edge or the disintegration tools or the counter-cutting edge.

11. Device according to claim 9, in which at least the separating edges or the disintegration tools are covered with synthetic diamond.

12. Device according to one of claims 1 to 19, in which a motor for rotating the cylindrical cutting body/milling body (54) is provided.

13. Device according to claim 12, in which the motor can rotate the cutting body/milling body (54) in both directions of rotation or can rotate it oscillatingly.

## Patentansprüche

1. Chirurgische Einhandvorrichtung mit einem Rohr (3) mit einer Spitze (5) an seinem vorderen Ende, wobei eine Vorrichtung zum Abtragen von Körpergewebe bei einem chirurgischen Eingriff als die Spitze verwendet wird, wobei die Vorrichtung aufweist:
ein Rohr (53) mit einer Längsachse, wobei das Rohr weiterhin einen Aspirationskanal (125) und eine Aspirationsöffnung (53b) zum Aufnehmen des abgetragenen Gewebes aufweist,
einen Schneid-/Fräskörper (54), der im Inneren des Rohrs so gelagert ist, dass er sich um eine Drehachse (56) drehen kann, wobei der Schneid-/Fräskörper (54) zumindest an einem Teil seines Umfangs eine Mehrzahl von Abtrennkanten (55) aufweist,
wobei der Schneid-/Fräskörper (54) so gelagert ist, dass die Abtrennkanten (55) nacheinander in der Aspirationsöffnung (53b) zu der Außenseite des Rohrs (53) so frei liegen, dass sie annähernd tangential zu der Aspirationsöffnung (53b) sind, wenn sich der Schneid-/Fräskörper (54) um die Drehachse (56) dreht,
wobei der Aspirationskanal (125) außerhalb des Schneid-/Fräskörpers (54) zu der Aspirationsöffnung (53b) geführt ist,
wobei die Spitze (5) mindestens eine Irrigationsflüssigkeitsaustrittsöffnung (5a) für den Austritt einer Irrigationsflüssigkeit aufweist,
wobei eine Irrigationsflüssigkeitsaustrittsöffnung (5a) für den Austritt der Irrigationsflüssigkeit in dem Rohr (53) der Vorrichtung zum Abtragen von Körpergewebe vorgesehen ist, und wobei die Einhandvorrichtung weiterhin aufweist
eine Irrigationsflüssigkeitspumpe (11), welche an dem Rohr (3) angeschlossen ist und mit der Irrigationsflüssigkeitsaustrittsöffnung (5a) über einen Irrigationskanal (115) verbunden ist,
eine Aspirationspumpe (12), welche an dem Rohr (3) angeordnet ist und mit der Aspirationsöffnung (5b) über den Aspirationskanal (125) verbunden ist, **dadurch gekennzeichnet, dass**
die Drehachse (56) bezüglich der Längsachse des Rohrs (53) verkippt ist oder senkrecht dazu ist,
an dem Irrigationskanal (115) ein Irrigationsflüssigkeitssensor (14) angeordnet ist,
an dem Aspirationskanal (125) ein Aspirationsflüssigkeitssensor (15) angeordnet ist, und
die Einhandvorrichtung eine Steuerung (13) aufweist, welche in Abhängigkeit von den Messresultaten der Sensoren (14, 15) den Augeninnendruck aktiv regelt.

2. Einhandvorrichtung nach Anspruch 1, bei der über einen optischen Lichtleiter Licht in die Spitze (5) führbar ist.

3. Einhandvorrichtung nach einem der Ansprüche 1 bis 2, bei der in der Spitze (5) eine Optik vorgesehen ist, die es erlaubt, das Operationsgebiet zu beobachten.

4. Einhandvorrichtung nach einem der Ansprüche 1 bis 3, bei der der Schneid-/Fräskörper (54) die Gestalt eines Prismas hat.

5. Einhandvorrichtung nach einem der Ansprüche 1 bis 4, bei der der Schneid-/Fräskörper (54) an der Umfangsfläche Schaufeln aufweist, die in der Drehrichtung gekrümmt sind, wobei die äußeren Kanten der Schaufeln die Abtrennkanten (55) bilden.

6. Vorrichtung nach einem der Ansprüche 1 bis 5, bei der die Abtrennkanten (55) parallel zu der Umfangsfläche des zylindrischen Schneid-/Fräskörpers (54) sind.

7. Vorrichtung nach einem der Ansprüche 1 bis 6, bei der zumindest eine Abtrennkante Zertrümmerungswerkzeuge aufweist.

8. Vorrichtung nach einem der Ansprüche 1 bis 7, bei der an dem Rand der Aspirationsöffnung (53b) eine Gegenschnittkante (7) ausgebildet ist.

9. Vorrichtung nach einem der Ansprüche 1 bis 8, bei der die Abtrennkante oder die Zertrümmerungswerkzeuge oder die Gegenschnittkante aus Edelstahl, Zirkon oder Keramikmaterial bestehen.

10. Vorrichtung nach Anspruch 9, bei der auf die Abtrennkante oder die Zertrümmerungswerkzeuge oder die Gegenschnittkante Diamantkristalle aufgewachsen sind.

11. Vorrichtung nach Anspruch 9, bei der zumindest die Abtrennkanten oder die Zertrümmerungswerkzeuge mit synthetischem Diamant überzogen sind.

12. Vorrichtung nach einem der Ansprüche 1 bis 11, bei der ein Motor zur Drehung des zylindrischen Schneid-/ Fräskörpers (54) vorgesehen ist.

13. Vorrichtung nach Anspruch 12, bei der der Motor den Schneid-/Fräskörper in beide Drehrichtungen oder oszillierend drehen kann.

## Revendications

1. Dispositif chirurgical à manier d'une main comprenant un tube (3) ayant une pointe (5) au niveau de son extrémité avant, dans lequel un dispositif pour retirer du tissu corporel dans une intervention chirurgicale est utilisé en tant que dite pointe,
ledit dispositif comprenant :
un tube (53) ayant un axe longitudinal, le tube ayant en outre un conduit d'aspiration (125) et une ouverture d'aspiration (53b) pour enlever le tissu retiré,
un corps de coupe/un corps de broyage (54) qui est monté à l'intérieur du tube de sorte qu'il peut tourner autour d'un axe de rotation (56), dans lequel le corps de coupe/corps de broyage (54) au moins au niveau d'une partie de sa périphérie, a une pluralité de bords de séparation (55),
dans lequel le corps de coupe/corps de broyage (54) est monté de sorte que les bords de séparation (55) sont successivement exposés dans l'ouverture d'aspiration (53b) vers l'extérieur du tube (53) de sorte qu'ils sont presque tangentiels à l'ouverture d'aspiration (53b), lorsque le corps de coupe/corps de broyage (54) tourne autour de l'axe de rotation (56),
dans lequel le conduit d'aspiration (125) fonctionne sur l'ouverture d'aspiration (53b) à l'extérieur du corps de coupe/corps de broyage (54),
dans lequel la pointe (5) a au moins une sortie d'orifice de fluide d'irrigation (5a) pour la sortie d'un fluide d'irrigation,
dans lequel une sortie d'orifice de fluide d'irrigation (5a) pour la sortie du fluide d'irrigation est prévue dans le tube (53) du dispositif pour retirer le tissu corporel et dans lequel le dispositif à manier d'une main comprend en outre :
une pompe de fluide d'irrigation (11) qui est fixée sur le tube (3) et est raccordée à la sortie d'orifice de fluide d'irrigation (5a) via un conduit d'irrigation (115),
une pompe d'aspiration (12) qui est agencée au niveau du tube (3) et est raccordée à l'ouverture d'aspiration (5b) via ledit conduit d'aspiration (125), **caractérisé en ce que** ledit axe de rotation (56) est incliné par rapport audit axe longitudinal du tube (53) ou perpendiculaire à celui-ci, et
un capteur de fluide d'irrigation (14) est agencé au niveau du conduit d'irrigation (115),
un capteur de fluide d'aspiration (15) est agencé au niveau du conduit d'aspiration (125), et
ledit dispositif à manier d'une main comprend une commande (13) qui régule activement la pression intraoculaire en fonction des résultats de mesure des capteurs (14, 15).

2. Dispositif à manier d'une main selon la revendication 1, dans lequel la lumière peut être guidée dans la pointe (5) via un guide de lumière optique.

3. Dispositif à manier d'une main selon l'une quelconque des revendications 1 à 2, dans lequel une optique est prévue dans la pointe (5) qui permet l'observation du site chirurgical.

4. Dispositif à manier d'une main selon l'une quelconque des revendications 1 à 3, dans lequel le corps de coupe/corps de broyage (54) a la forme d'un prisme.

5. Dispositif selon l'une quelconque des revendications 1 à 4, dans lequel le corps de coupe/corps de broyage (54) a des pales au niveau de la surface périphérique qui sont incurvées dans la direction de rotation, dans lequel les bords externes des pales forment les bords de séparation (55).

6. Dispositif selon l'une quelconque des revendications 1 à 5, dans lequel les bords de coupe (55) sont parallèles à la surface périphérique du corps de coupe/corps de broyage cylindrique (54).

7. Dispositif selon l'une quelconque des revendications 1 à 6, dans lequel au moins un bord de séparation a des outils de désintégration.

8. Dispositif selon l'une quelconque des revendications 1 à 7, dans lequel à la bordure de l'ouverture d'aspiration (53b), est formé un bord de contre-coupe (7).

9. Dispositif selon l'une quelconque des revendications 1 à 8, dans lequel le bord de séparation ou les outils de désintégration ou le bord de contre-coupe est/sont réalisé(s) à partir d'acier inoxydable, de zircon ou de matériau en céramique.

10. Dispositif selon la revendication 9, dans lequel des cristaux de diamant font saillie sur le bord de séparation ou les outils de désintégration ou le bord de contre-coupe.

11. Dispositif selon la revendication 9, dans lequel au moins les bords de séparation ou les outils de désintégration sont recouverts avec le diamant synthétique.

12. Dispositif selon l'une quelconque des revendications 1 à 11, dans lequel on prévoit un moteur pour faire tourner le corps de coupe/corps de broyage cylindrique (54).

13. Dispositif selon la revendication 12, dans lequel le moteur peut faire tourner le corps de coupe/corps de broyage (54) dans les deux directions de rotation ou peut tourner sur lui-même de manière oscillante.
